# EUROPEAN PATENT SPECIFICATION

(11) **EP 2 768 416 B1**
(45) Date of publication and mention of the grant of the patent: **16.03.2016**
(21) Application number: 12855412.8
(22) Date of filing: 26.11.2012
(51) Int. Cl.: A61F 9/008

(54) **DEVICES AND METHODS FOR RECONFIGURABLE MULTISPOT SCANNING**
VORRICHTUNGEN UND VERFAHREN FÜR REKONFIGURIERBARE MEHRPUNKT-ABTASTUNG
DISPOSITIFS ET PROCÉDÉS DE BALAYAGE MULTIPOINT RECONFIGURABLE

(30) Priority: 09.12.2011 US 201161568695 P
(43) Date of publication of application: 27.08.2014
(73) Proprietor: Alcon Research, Ltd., Fort Worth TX 76134 (US)
(72) Inventor: SMITH, Ronald, T., Irvine, California 92618 (US); DACQUAY, Bruno, Irvine, California 92603 (US)
(74) Representative: Hanna, Peter William Derek
(86) International application number: PCT/US2012/066538
(87) International publication number: WO 2013/085736

(56) References cited:
- WO-A1-2009/009246
- US-A1- 2003 231 827
- US-A1- 2006 217 695
- US-A1- 2007 265 602
- US-A1- 2008 015 553
- US-A1- 2008 080 206
- US-A1- 2009 257 065
- US-A1- 2011 116 040
- US-A1- 2011 144 627
- US-B2- 7 648 242
- JELINKOVA H. ET AL.: 'Hollow waveguide delivery systems for laser technological application' PROGRESS IN QUANTUM ELECTRONICS, [Online] vol. 28, 2004, pages 145 - 164, XP004548754 Retrieved from the Internet: <URL:http://www.sciencedirect.com/science/a rticle/pii/S0079672703000995> [retrieved on 2013-01-16]

## Description

### FIELD

The present application relates to a probe for use in ophthalmic procedures and more particularly to a multispot laser probe for use in photocoagulation.

### BACKGROUND

Laser photocoagulation therapy addresses ocular conditions such as retinal detachments and tears as well as proliferative retinopathy resulting from diseases such as diabetes. The abnormally high blood sugar in a diabetic stimulates the retinal vessels to release growth factors that in turn encourage an undesirable proliferation of blood vessels and capillaries over the retinal surface. These proliferated blood vessels are very delicate and will readily bleed into the vitreous. The body responds to the damaged vessels by producing scar tissue, which may then cause the retina to detach so as to eventually cause blindness.

In laser photocoagulation, a laser probe is used to cauterize the blood vessels at various laser burn spots across the retina. Because the laser will also damage the rods and cones that are present in the retina to allow vision, eyesight, as well as the blood vessels, is affected. Since vision is most acute at the central macula of the retina, the surgeon arranges the resulting laser burn spots in the peripheral areas of the retina. In this fashion, some peripheral vision is sacrificed to preserve central vision. During the procedure, the surgeon drives the probe with a non-burning aiming beam such that the retinal area to be photocoagulated is illuminated. Due to the availability of low-power red laser diodes, the aiming beam is generally a low-power red laser light. Once the surgeon has positioned the laser probe so as to illuminate a desired retinal spot, the surgeon activates the laser through a foot pedal or other means to then photocoagulate the illuminated area. Having burned a retinal spot, the surgeon repositions the probe to illuminate a new spot with the aiming light, activates the laser, repositions the probe, and so on until a suitable array of burned laser spots are distributed across the retina.

The number of required laser photocoagulations for any one treatment of the retina is large--for example, 1,000 to 1,500 spots are commonly burned. It may thus be readily appreciated that if the laser probe was a multi-spot probe enabling the burning of multiple spots with each position of the probe, the photocoagulation procedure would be faster (assuming the laser source power is sufficient). Existing multi-spot probes may be inadequate in some respects and thus, there is a need in the art for improved multi-spot laser probes.

US 2007/0265602 A1 teaches an ophthalmic laser probe system without an adapter having movable reflective surfaces therein.

### SUMMARY

Further aspects, forms, embodiments, objects, features, benefits, and advantages of the present invention shall become apparent from the detailed drawings and descriptions provided herein.

In one embodiment, an ophthalmic laser probe system comprises an array of optical waveguides and an adapter operable to connect with a laser source. The laser probe system further includes a first reflective surface within the adapter. The first reflective surface is movable about a first axis. The laser probe system also includes a second reflective surface within the adapter. The second reflective surface is movable about a second axis, orthogonal to the first axis. The first reflective surface is configured to receive a laser beam emitted from the laser source and redirect the laser beam toward the second reflective surface.

Also disclosed is a method of laser photocoagulation comprises providing an adapter for connecting a laser source to array of optical waveguides and directing a laser beam from the laser source toward a first reflective surface arranged in a first configuration. The method also includes receiving the laser beam reflected from the first reflective surface at a second reflective surface and directing the laser beam from the second reflective surface to a first optical waveguide in the array of optical waveguides.

In still another embodiment, a laser probe adapter comprises a first connector portion sized and configured for connection to a subminiature version A (SMA) connector on a laser source. The adapter further includes a second connector portion sized and configured for connection to an array of optical waveguides. The adapter further includes a first reflective surface movable about a first axis and a second reflective surface movable about a second axis orthogonal to the first axis. The first reflective surface is configured to receive a laser beam emitted from the laser source and redirect the laser beam toward the second reflective surface.

### BRIEF DESCRIPTION OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In addition, the present disclosure may repeat reference numerals and/or letters in the various examples. This repetition is for the purpose of simplicity and clarity and does not in itself dictate a relationship between the various embodiments and/or configurations discussed.
Fig. 1 is an longitudinal cross-sectional view of an adapter, according to one embodiment of this disclosure, coupled between a laser source and an optical connector.
Fig. 2 is a partially exploded perspective view of an adapter and an optical connector, according to another embodiment of this disclosure.
Fig. 3 is an assembled perspective view of the adapter and optical connector of Fig. 2.
Fig. 4 is a side view of an optical assembly according to one embodiment of the present disclosure.
Fig. 5 is a bottom view of the optical assembly in the embodiment of Fig. 4.
Fig. 6 is a radial cross sectional view of a secondary gradient index (GRIN) lens in the optical assembly of the embodiment of Fig. 4.
Fig. 7 is a side view of an optical assembly of Fig. 4 in a first configuration.
Fig. 8 is a bottom view of the optical assembly of Fig. 4 in the first configuration.
Fig. 9 is a radial cross sectional view of a secondary GRIN lens in the optical assembly of Fig. 4 in the first configuration.
Figs. 10a, 10b, 10c, and 10d are radial cross sectional views of a multi-fiber array with an optical assembly in four different configurations according to an embodiment of the present disclosure.
Fig. 11 is a view of a laser beam spot on an image plane according to one embodiment of the present disclosure.
Figs. 12a, 12b, 12c, 12d are different configurations of multiple laser beam spots on an image plane according to the embodiment of Fig. 11.
Fig. 13 is a view of a laser beam spot on an image plane according to another embodiment of the present disclosure.
Figs. 14a, 14b, 14c, 14d, 14e, and 14f are different configurations of multiple laser beam spots on an image plane according to the embodiment of Fig. 13.
Fig. 15 is a method for operating a laser probe according to an embodiment of this disclosure.

### DETAILED DESCRIPTION OF EMBODIMENTS

For the purposes of promoting an understanding of the invention, reference will now be made to the embodiments, or examples, illustrated in the drawings and specific language will be used to describe the same. It will nevertheless be understood that no limitation of the scope of the invention is thereby intended.

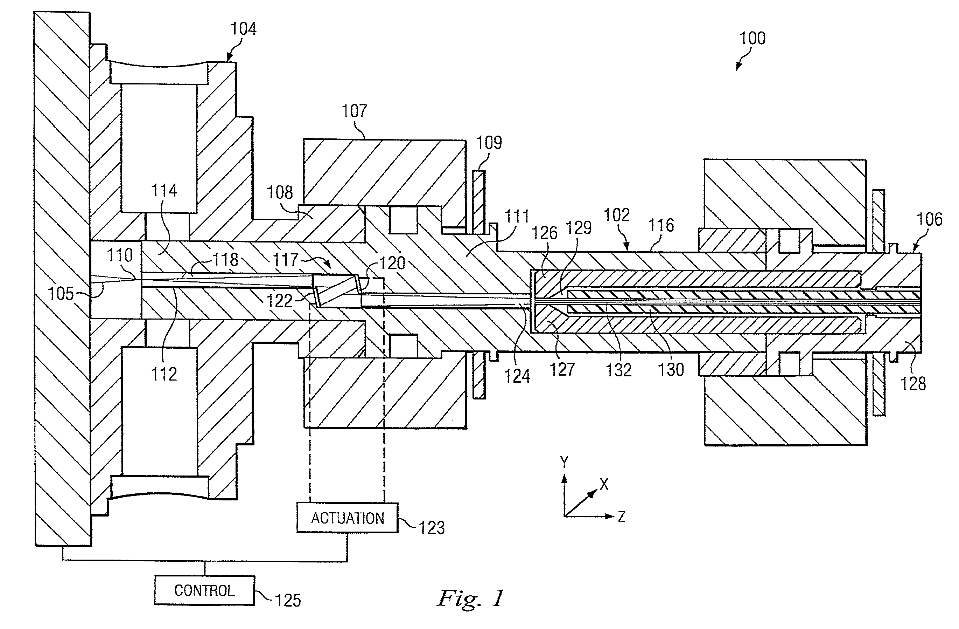

Fig. 1 is a longitudinal cross-sectional view of a laser probe assembly 100 including an adapter 102, according to one embodiment of this disclosure, coupled between a laser source 104 and a optical connector 106. The assembly 100 may be used for intraocular treatments, including for example, ophthalmic photocoagulation. The laser source 104 generates a laser beam 105 and includes a common standardized interconnect 108 which may be a subminiature version A (SMA) adapter. Although the interconnect 108 is depicted as a female SMA adapter, it will be appreciated that any conventional standardized optical interconnect may be used so long as the laser source's interconnect presents a focused beam spot such as a laser waist 110 to the proximal end of the adapter 102. A suitable laser source 104 may be a Purepoint® Laser provided by Alcon Laboratories, Inc. of Ft. Worth, Texas. In one example, the laser beam 105 includes a green treatment laser beam, propagating with a wavelength of approximately 532 nanometers (nm). An aiming beam, such as a red beam, may be transmitted before, after, or with the treatment laser beam to assist the ophthalmic surgeon with targeting the treatment laser beam.

The adapter 102 includes an adapter body 111 in which a bore 112 extends. In this embodiment, the adapter body 111 includes a proximal male connector 114 and a distal female connector 116. The male connector 114 may be sized and shaped to connect with the interconnect 108, and thus may be, for example, a male SMA connector. The female connector 116 may be sized and shaped to interconnect with the optical connector 106. It is understood that the male and female configurations of the adapter connectors are merely examples and other types of known interconnect formations may be suitable. In this embodiment, a ring connector 107 threadedly connects with the interconnect 108 to secure the adapter 102 to the laser source 104. A retaining ring 109 retains the ring connector 107, preventing it from slipping distally off of the adapter housing 111.

The adapter 102 further includes an optical assembly 117 which includes a collimating lens 118, such as a GRIN lens, disposed in the bore 112 to receive and collimate the laser beam 105. The adapter 102 also includes a movable reflective device 120 to redirect the collimated laser beam 105. The adapter 102 also includes a movable reflective device 122 to redirect the collimated laser beam 105. Each of the reflective devices 120, 122 may be, for example, a micro-electomechanical mirror that is pivotable between one or more positions. The adapter 102 also include a focusing lens 124, such as a GRIN lens, which receives and focuses the laser beam 105. Movement of the reflective devices 120, 122 allows the focused laser beam 105 to be moved in two dimensions on an image plane, as will be discussed in greater detail below.

An actuation system 123 operates the movable reflective devices 120, 122. The actuation system 123 includes electrical wires to deliver power to motors which operate the devices 120, 122. Portions of the actuation system 123 may be located in the adapter 102. The actuation system 123 is controlled by a control system 125. The control system 125, such as a laptop computer running control software, is also connected to the laser source 104. The control system 125 may be used to synchronize the laser with the motion of the reflective devices 120, 122. For example, the laser 104 may be blocked when the reflective devices 120, 122 are in motion and unblocked when the reflective devices are stopped. Alternatively the laser 104 may be pulsed when the reflective devices 120, 122 are stopped and not pulsed when the reflective devices are in motion. Other techniques to generate an on/off pattern for the laser, as are known in the art, may be used to synchronize the laser with the scanning of the reflective devices.

The optical connector 106 has a proximal end 126 inserted into the female connector 116 of the adapter 102. The optical connector 106 may be, for example, a straight tip (ST) connector to permit rotational alignment between adapter 102 and an array of waveguides in the optical connector 106. The proximal end 126 of the optical connector 106 is separated from the distal end of the GRIN lens 124 by a suitable air gap such as a 220 micrometer (µm) air gap. The optical connector 106 includes ferrule 127 inserted into a connector body 128. The ferrule 127 and the connector body 128 may be formed of a relatively rigid material such as stainless steel. The ferrule 127 includes a tapered passage 129 through which an array of optical waveguides 132 extend. The array of optical waveguides 132 extends from the ferrule 127 through the connector body 128 and to a handpiece and cannula, as known in the art of laser probes. Optionally, a cylindrical sleeve 130 encloses the array of optical waveguides 132. The cylindrical sleeve 130 may be formed of a relatively flexible material, such as a polymer material. In this embodiment, the plurality of optical waveguides are an array of four optical fibers. In alternative embodiments, arrays of different sizes or different wave transmission structures may be used. In one embodiment, the array of optical fibers may comprise four 75 µm core glass fibers, each with a numerical aperture (NA) of 0.22. The optical waveguides, the cannula, and/or the handpiece may be disconnected from the adapter and disposed of after a single use, allowing the adapter to be reused for subsequent procedures. During procedures using the laser probe assembly 100, the adapter 106 may be located within or outside of the sterile field. If the adapter is located within the sterile field it may remain sterilized through multiple procedures or, alternatively, may be sterilized after each procedure.

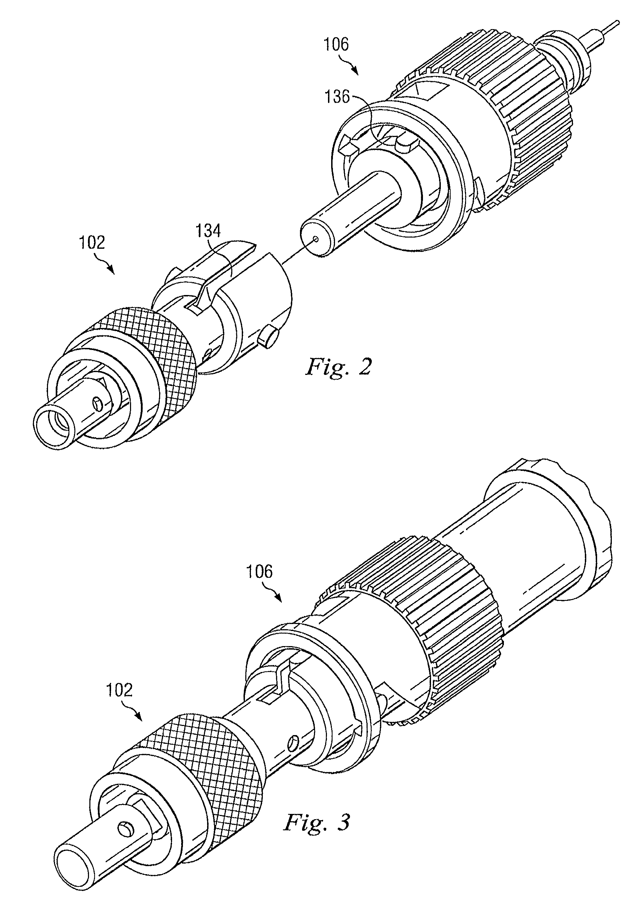

Figs. 2 and 3 depict, respectively, partially exploded and assembled perspective views of the adapter 102 and a optical connector 106. As shown, the proximal end 126 of the optical connector 106 is inserted into the female connector 116 of the adapter 102. Alignment features, such as the shown slot 134 and key 136, may be used for the clocking or rotational alignment of the optical connector 106 with the adapter 102.

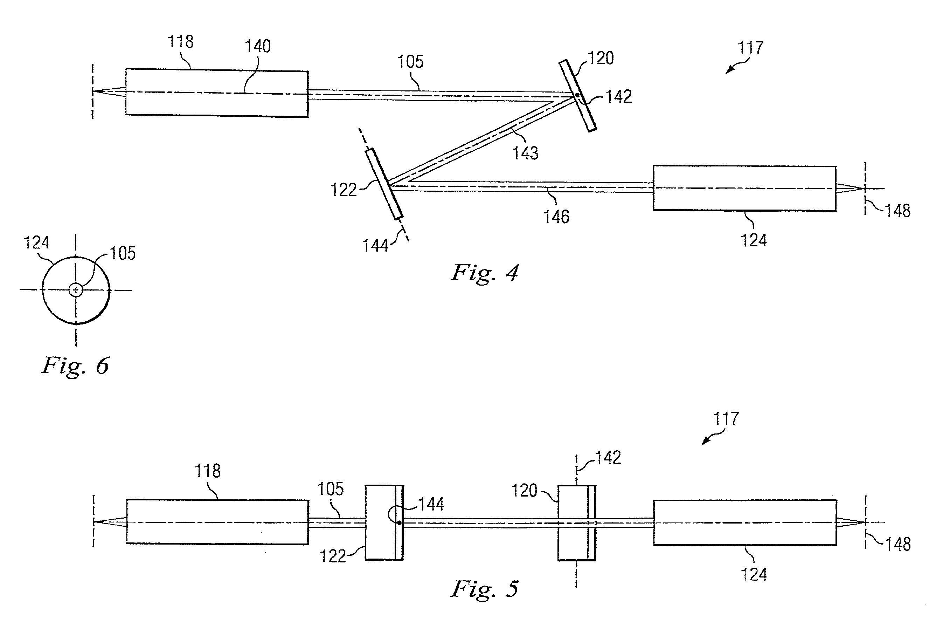

Fig. 4 is a side view and Fig. 5 is a bottom view of the optical assembly 117 with the reflective devices 120, 122 configured to deliver the laser beam 105 to a central location. The laser beam 105 propagates through the GRIN lens 118 along an axis 140 to the reflective device 120. In this embodiment, the reflective device 120 is pivotable about an axis 142, normal to the Fig. 4 plane. The reflective device 120 redirects the laser beam 105 to the reflective device 122 along a path 143. In this embodiment, the reflective device 122 pivots about an axis 144, which is orthogonal to the axis 142. The reflective device 122 redirects the laser beam 105 toward the GRIN lens 124 along an axis 146. The collimated laser beam 105 is incident upon the GRIN lens 124 at a normal incident angle, and the GRIN lens focuses the laser beam to a tiny spot along the axis 146. In this configuration of the reflective devices 120, 122, the laser beam 105 is centrally aligned with the axis 146 and impacts a coupling plane 148 at a central location. Fig. 6 is a radial cross sectional view of the GRIN lens 124 as viewed looking backward from the coupling plane 148 towards GRIN lens 124, with the reflective devices 120, 122 in the configuration of Figs. 4 and 5.

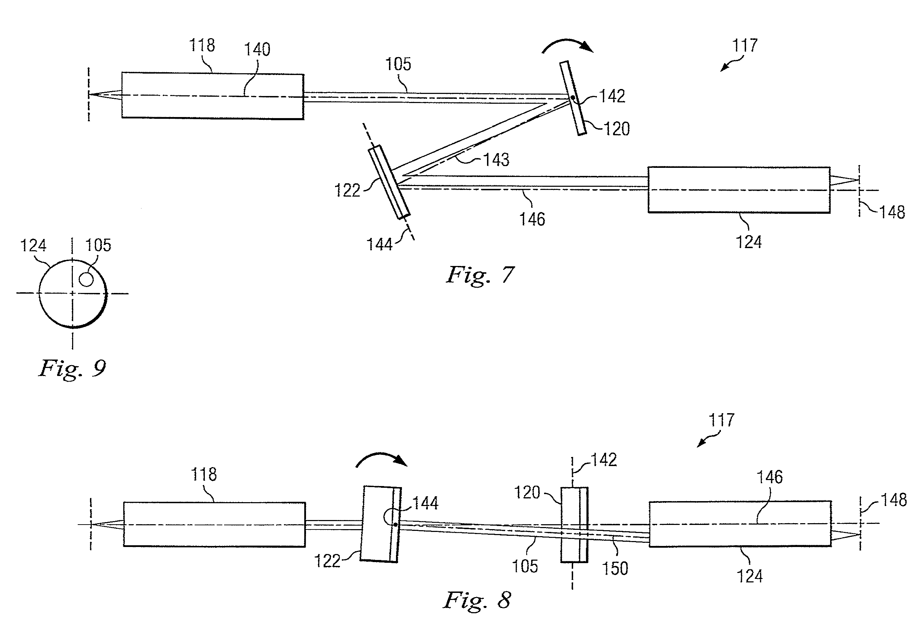

Fig. 7 is a side view and Fig. 8 is a bottom view of the optical assembly 117 with the reflective devices 120, 122 configured to deliver the laser beam 105 to a location shifted above and to the right of the central location in Figs 4-6 (as viewed looking backward from the coupling plane 148 towards GRIN lens 124). The laser beam 105 propagates through the GRIN lens 118 along an axis 140 to the reflective device 120. In this embodiment, the reflective device 120 is pivoted clockwise about the axis 142. The pivoted position of the reflective device 120 redirects the laser beam 105 to the reflective device 122 along a path shifted upward from path 143. In this embodiment, the reflective device 122 is pivoted clockwise about the axis 144 (as viewed in Fig. 8). The pivoted position of the reflective device 122 redirects the laser beam 105 toward the GRIN lens 124 along a path 150 shifted right relative to the axis 146. In this configuration of the reflective devices 120, 122, the laser beam 105 impacts a coupling plane 148 at a location shifted above and to the right of the central location in Figs. 4-6 (as viewed looking backward from the coupling plane 148 towards GRIN lens 124). Fig. 9 is a radial cross sectional view of the GRIN lens 124 as viewed looking backward from the coupling plane 148 towards the GRIN lens 124, with the reflective devices 120, 122 in the configuration of Figs. 7 and 8. As shown, the beam 105 is positioned in an upper right quadrant of the lens 124.

The optical assembly 117, as described, provides a predictable mechanism for directing laser light in two dimensions by using two reflective devices 120, 122 that are angularly adjusted about axes that are orthogonal to one another. In alternative embodiments, a single reflective device pivotable about one or more axes may be used to generate an array of laser beam spots. In still another alternative, more than two reflective devices pivotable about one or more axes may be used to generate an array of laser beam spots. In still another embodiment, one or more of the reflective devices may be fixed while other reflective devices may be movable. In still another embodiment, a single reflective device can be rotated to approximately 45° relative to the beam exiting the first GRIN lens, directing the beam into a second GRIN lens positioned substantially orthogonal to the first GRIN lens. In this case, the fiber array would be orthogonal to the initial axis of the beam.

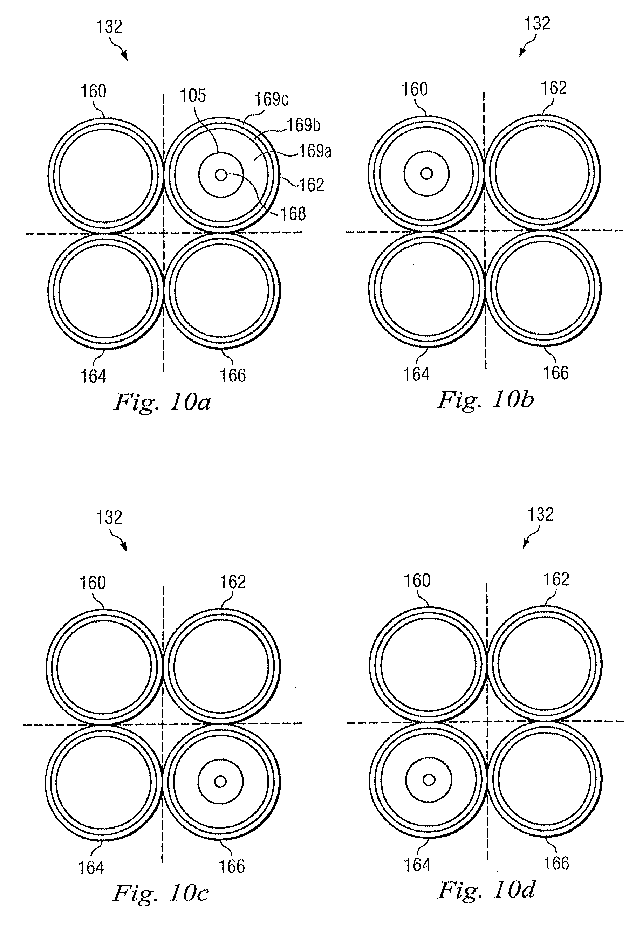

Figs. 10a, 10b, 10c, and 10d are radial cross sectional views of the fiber array 132 looking backward toward the GRIN lens 124. These figures depict the fiber array 132 with an optical assembly 117 in four different configurations. The fiber array 132 includes optical fibers 160, 162, 164, 166. In Fig. 10a, the reflective devices 120, 122, in the configuration of Figs. 7-9 shift the laser beam 105 and an aiming beam 168 so that the beams couple with the upper right optical fiber 162. In Fig. 10b, the reflective device 120 is held in the same position as in Figs. 7-9, but reflective device 122 is pivoted counterclockwise to direct the beams 105, 168 leftward. With the reflective devices 120, 122 in this configuration, the beams 105, 168 couple with the upper left optical fiber 160. In Fig. 10c, the reflective device 122 is held in the same position as in Figs. 7-9, but reflective device 120 is pivoted counterclockwise to direct the beams 105, 168 downward. With the reflective devices 120, 122 in this configuration, the beams 105, 168 couple with the lower right optical fiber 166. In Fig. 10d, the reflective device 120 is held in the same position as in Figs. 10c, but reflective device 122 is pivoted counterclockwise to direct the beams 105, 168 leftward. With the reflective devices 120, 122 in this configuration, the beams 105, 168 couple with the lower left optical fiber 164. With each configuration of the optical assembly 117, both the laser beam 105 and aiming beam 168 may be spatially located on the centers of the cores of each of the optical fibers 160, 162, 164, 166. Each of the optical fibers 160, 162, 164, 166 includes a core 169a, a cladding 169b surrounding the core, and a jacket 169c surrounding the core. The beams 105, 168 are transmitted through the core 169a. The depicted core, cladding, and jacket are not to scale, and each may be formed with a suitable diameter as is known in the art.

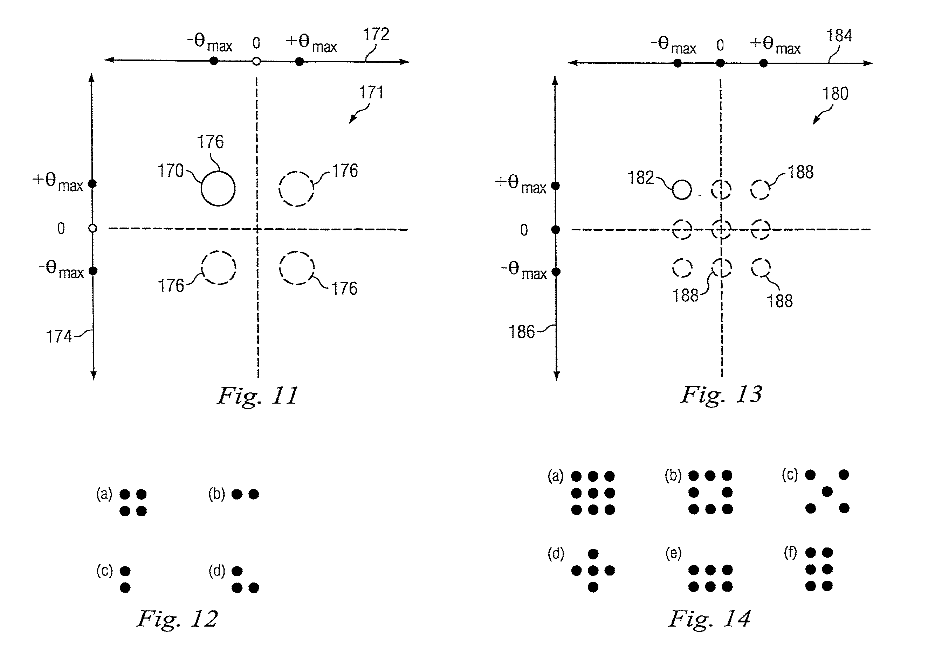

Thus, as demonstrated in Figs. 10a-10d, reflective device 120 controls the vertical position of the laser beam and reflective device 122 controls the horizontal position of the laser beam. Fig. 11 is a view of a laser spot 170 generated by laser beam 105 on an image plane 171, which may correspond to the retina. The laser spot 170 is generated by the optical assembly 117 with the reflective devices 120, 122, in the configuration of Figs. 7, 8, and 9. Each reflective device has an angular range of travel with hard stops at the ends of the range. The range of motion 172 of reflective device 122 has stop positions at +/-θₘₐₓ. The range of motion 174 of reflective device 120 has stop positions at +/-θ_{max.} These stop positions may be highly repeatable positions. Actuating each of the reflective devices 120, 122 to move from +θₘₐₓ to - θ_{max,} allows four laser spots to be generated on the image plane 171 in any of four locations 176 in a 2x2 array. The laser source 104 is synchronized with the movement of the reflective devices 120, 122 so that the laser beam can be blocked during the inter-spot transits and can be unblocked during dwell times at each location 176. The reflective devices 120, 122 may be controlled so that the scanning laser beam 105 cycles rapidly between the spots 176 and remains on each spot for a prescribed dwell time to create a spot pattern. Many beam scan cycles can be performed in a second, thus allowing a retina to be treated with a pattern of burn spots quickly. For example, a four scan cycle, with each cycle having approximately a 50 millisecond (msec.) duration, may result in a total burn time of approximately 200 msec. The optical assembly 117 thus allows an ophthalmic surgeon to deliver multiple burns without moving the laser probe.

As shown in Figs. 12a-d, different burn patterns may be created with the reflective devices 120, 122 at hard stops +θₘₐₓ or -θₘₐₓ. Fig. 12a depicts a 2x2 pattern on the image plane. Fig. 12b depicts a horizontal 2x1 pattern on the image plane. Fig. 12c depicts a vertical 1x2 pattern on the image plane. Fig. 12d depicts a triangular pattern on the image plane. Thus, the optical assembly 117 allows for reconfigurable laser beam positions and resulting burn patterns. The reconfiguration of the burn pattern may be made, real time, by the control system 125. As would be understood, other patterns may be generated by reducing one or more spots in the 2x2 pattern. Further, the dwell time for each spot in a burn pattern can be reconfigured to give spatially non-uniform exposures. Such non-uniform exposures may be used, for example, to compensate for the fact that spots travelling the furthest to the tissue site or an off-axis titled laser probe are larger, and therefore have less irradiance than the near spots.

In an alternative embodiment, the reflective devices 120, 122 may be controlled to stop at one or more angular tilt positions along the continuum between +/-θₘₐₓ. For example, as shown in Fig. 13, the optical assembly 117 may be controlled to accurately pivot each of the reflective devices 120, 122 to an intermediate location 0, midway between +θₘₐₓ and -θₘₐₓ. In this embodiment, a 3x3 array of optical fibers are spatially aligned to couple with each of the nine possible paths of the laser beam. Fig. 13 shows an image plane 180 with a laser spot 182 generated by laser beam 105. Each reflective device 120, 122 has an angular range of travel with two hard stop positions at either end of the range and an intermediate stop position located between the hard stop positions. The range of motion 184 of reflective device 122 has stop positions at +θₘₐₓ, 0, and -θₘₐₓ. The range of motion 186 of reflective device 120 has stop positions at +θₘₐₓ, 0, and -θₘₐₓ. These stop positions are highly repeatable positions, although greater control may be required to accurately repeat position 0 as compared to the hard stop positions at the end of the angular range. Actuating each of the reflective devices 120, 122 to move between stop positions +θₘₐₓ, 0, -θₘₐₓ, allows nine laser spots 188 to be generated on the image plane 180. The laser source 104 is synchronized with the movement of the reflective devices 120, 122 so that the laser beam can be blocked during the inter-spot transits and can be unblocked during dwell times at each location 188. The reflective devices 120, 122 may be controlled so that the scanning laser beam 105 cycles rapidly between the spots 188 and remains on each spot for a prescribed dwell time to create a spot pattern.

As shown in Figs. 14a-f, different burn patterns may be created with the reflective devices 120, 122 at stop positions +θₘₐₓ, 0, and -θₘₐₓ. Fig. 14a depicts a 3x3 pattern on the image plane. Fig. 14b depicts a 3x3 open box pattern on the image plane. Fig. 14c depicts an X pattern on the image plane. Fig. 14d depicts a + pattern on the image plane. Fig. 14e depicts a 3x2 pattern on the image plane. Fig. 14f depicts a 2x3 pattern on the image plane. Thus, the optical assembly 117 allows for reconfigurable laser beam positions and resulting burn patterns. As would be understood, other patterns may also be generated by reducing one or more spots in the 3x3 pattern.

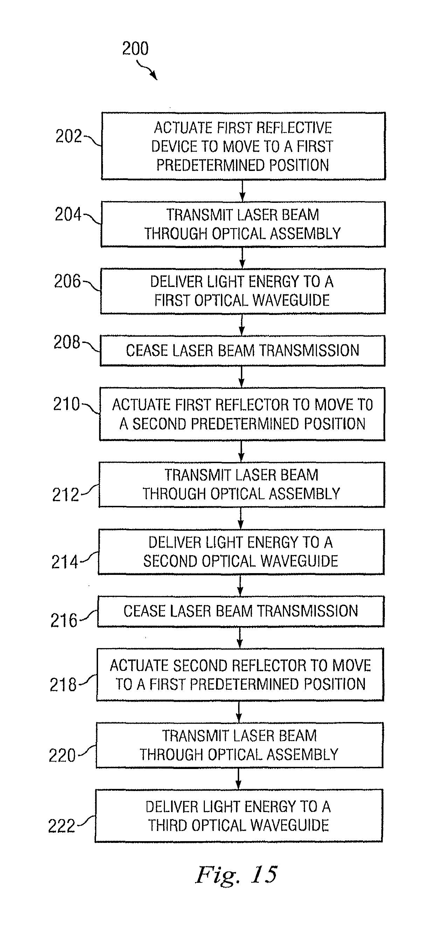

Referring now to Fig. 15, a method for operating the laser probe assembly 100 includes the step 202 of actuating reflective device 120 to move to stop position +θₘₐₓ while the reflective device 122 is positioned at stop position -θₘₐₓ. At step 204, the laser beam 105 is transmitted through the optical assembly 117 by unblocking a continuous laser beam or pulsing a laser beam. At step 206, the laser beam 105 is coupled to optical fiber 162 for delivery to a tissue location 170. At step 208, the transmission of the laser beam 105 is ceased, for example, by blocking the laser beam or turning it off. At step 210, the reflective device 120 is actuated to move to stop position -θₘₐₓ while the reflective device 122 remains positioned at stop position - θₘₐₓ. At step 212, the laser beam 105 is transmitted through the optical assembly 117. At step 214, the laser beam 105 is coupled to optical fiber 166 for delivery to a location below location 170. At step 216, the transmission of the laser beam 105 is ceased, for example, by blocking the laser beam or turning it off. At step 218, the reflective device 120 is held at stop position -θₘₐₓ and reflective device 122 is actuated to move to stop position +θₘₐₓ. At step 220, the laser beam 105 is transmitted through the optical assembly 117. At step 222, the laser beam 105 is coupled to optical fiber 164 for delivery to a third tissue location. To generate other patterns, the order of moving the reflective devices may be varied. Both reflective devices or only one reflective device may be moved between a laser pulse.

As compared to laser probe systems that produce only a single laser beam, the embodiments of this disclosure may increase the number of locations at a target tissue site that may be treated while decreasing the time to treat the multiple locations. Further, more complicated scan patterns may be generated. The embodiments described permit reconfiguration of the scan pattern in real time. Compared to diffraction-based beam splitting multi-spot laser probes that may deliver 70% or less of the laser beam to the optical fibers and then divide the beam among the multiple optical fiber, the embodiments of the present disclosure are more efficient and deliver more power to the tissue site with each scan of the laser probe. The embodiments of this disclosure may have near 100% efficiency. In one embodiment, with a 2 Watt (W) laser at nearly 100% efficiency, approximately 250 milliwatt (mW) may be delivered to each spot in a nine-spot pattern, enough to yield adequate burns for some applications. For example, a laser calibrated to deliver 2 Watts (2000 milliwatts) out of the distal end of a conventional 90% optical transmittance probe may provide an input beam 105 of 2222 milliwatts (i.e., 2000/0.9) to compensate for the optical loss in the probe. Since, in some embodiments, the optical transmittance of the laser probe assembly 100 may be near 100%, that same input laser beam 105 (2222 milliwatts) may provide an average power for each spot of a nine spot pattern of approximately 2222/9 = 246.9 milliwatts. In other words, while the laser beam leaving the probe assembly 100 is directed at a particular spot in the pattern, the instantaneous power on the spot may be 2222 milliwatts (in this example), but the average power into each beam spot may be calculated as the energy applied to a particular spot divided by the entire time it takes to make the pattern (which, in this example, results in an average power of approximately 246.9 milliwatts per spot). As further compared to diffraction-based multi-spot laser probes, coupling both a red aiming beam and a green treatment beam, which diffract at different angle, to a common optical fiber is simplified using the embodiments as disclosed.

In various embodiments, a laser probe adapter may include a first connector portion sized and configured for connection to a subminiature version A (SMA) connector on a laser source, a second connector portion sized and configured for connection to an array of optical waveguides, a first reflective surface movable about a first axis, and a second reflective surface movable about a second axis substantially orthogonal to the first axis (the first reflective surface may be configured to receive a laser beam emitted from the laser source and redirect the laser beam toward the second reflective surface). In some embodiments, the laser probe adapter may further include a micro-electromechanical mirror which includes the first reflective surface. In some embodiments, the laser probe adapter may further include a gradient index lens configured to receive the laser beam emitted from the laser source and direct the laser beam toward the first reflective surface. In some embodiments, the laser probe adapter may further include a gradient index lens configured to receive the laser beam reflected from the second reflective surface. In some embodiments, the first reflective surface may have a first configuration for directing the laser beam to a first optical waveguide in the array of optical waveguides. In some embodiments, the first reflective surface may have a second configuration for directing the laser beam to a second optical waveguide in the array of optical waveguides. In some embodiments, the first reflective surface may have a third configuration for directing the laser beam to a third optical waveguide in the array of optical waveguides.

In various embodiments, an ophthalmic laser probe system may include an array of optical waveguides, an adapter operable to connect with a laser source, a first gradient index lens within the adapter, a second gradient index lens within the adapter positioned substantially orthogonal to the first gradient index lens, a first reflective surface within the adapter, disposed along an optical path between the first and second gradient lenses and configured direct a laser beam received from the first gradient index lens toward the second gradient index lens.

The term "such as," as used herein, is intended to provide a non-limiting list of exemplary possibilities. The term "approximately" or "about," as used herein, should generally be understood to refer to both numbers in a range of numerals. Moreover, all numerical ranges herein should be understood to include each whole integer and tenth of an integer within the range. It is understood that all spatial references, such as "upper," "lower," "above," and "below" are for illustrative purposes only and can be varied within the scope of the disclosure.

While various embodiments of the invention have been described above, it should be understood that they have been presented by way of example only, and not limitation.

## Claims

1. An ophthalmic laser probe system comprising:
an array of optical waveguides (132);
an adapter (102) operable to connect with a laser source (104);
a first reflective surface (120) within the adapter, the first reflective surface movable about a first axis; and
a second reflective surface (122) within the adapter, the second reflective surface movable about a second axis substantially orthogonal to the first axis, wherein the first reflective surface (120) is configured to receive a laser beam (105) emitted from the laser source and redirect the laser beam toward the second reflective surface (122).

2. The laser probe system of claim 1, further including a micro-electromechanical mirror which includes the first reflective surface (120).

3. The laser probe system of claim 1, further comprising a gradient index lens (118) within the adapter (102), the gradient index lens configured to receive the laser beam (105) emitted from the laser source and direct the laser beam toward the first reflective surface (120).

4. The laser probe system of claim 1, further comprising a gradient index lens (124) within the adapter (102), the gradient index lens configured to receive the laser beam (105) reflected from the second reflective surface (122).

5. The laser probe system of claim 1, wherein the first reflective surface (120) has a first configuration for directing the laser beam (105) to a first optical waveguide (160) in the array of optical waveguides (132).

6. The laser probe system of claim 5, wherein the first reflective surface (120) has a second configuration for directing the laser beam (105) to a second optical waveguide (162) in the array of optical waveguides (132).

7. The laser probe system of claim 6, wherein the first reflective surface (120) has a third configuration for directing the laser beam (105) to a third optical waveguide (164) in the array of optical waveguides (132).

## Patentansprüche

1. Ophthalmische Lasersondensystem, aufweisend:
ein Array optischer Wellenleiter (132);
ein Adapter (102), der zum Anschließen an eine Laserquelle (104) konzipiert ist;
eine erste reflektierende Oberfläche (120) innerhalb des Adapters, wobei die erste reflektierende Oberfläche um eine erste Achse bewegbar ist; und
eine zweite reflektierende Oberfläche (122) innerhalb des Adapters, wobei die zweite reflektierende Oberfläche um eine zweite Achse bewegbar ist, die im Wesentlichen senkrecht auf die erste Achse steht,
wobei die erste reflektierende Oberfläche (120) konfiguriert ist, einen Laserstrahl (105) zu empfangen, der von der Laserquelle emittiert wird, und zum Rückführen des Laserstrahls in Richtung zu der zweiten reflektierenden Oberfläche (122).

2. Lasersondensystem nach Anspruch 1, ferner aufweisend einen mikroelektromechanischen Spiegel, der die erste reflektierende Oberfläche (120) aufweist.

3. Lasersondensystem nach Anspruch 1, ferner aufweisend eine Gradienten-Indexlinse (118) innerhalb des Adapters (102), wobei die Gradienten-Indexlinse
dazu konfiguriert ist, den Laserstrahl (105) zu empfangen, der von der Laserquelle emittiert wird, und diesen Laserstrahl in Richtung auf die erste reflektierende Oberfläche (120) zu richten.

4. Lasersondensystem nach Anspruch 1, ferner aufweisend eine Gradienten-Indexlinse (124) innerhalb des Adapters (102), wobei die Gradienten-Indexlinse dazu konfiguriert ist, den Laserstrahl (105) zu empfangen, der von der zweiten reflektierenden Oberfläche (122) reflektiert wird.

5. Lasersondensystem nach Anspruch 1, wobei die erste reflektierende Oberfläche (120) eine erste Konfiguration hat zum Ausrichten des Laserstrahls (105) an eine erste optische Wellenführung (160) in dem Array von optischen Wellenführungen (132).

6. Lasersondensystem nach Anspruch 5, wobei die erste reflektierende Oberfläche (120) eine zweite Konfiguration hat zum Ausrichten des Laserstrahls (105) an eine zweite optische Wellenführung (162) in dem Array von optischen Wellenführungen (132).

7. Lasersondensystem nach Anspruch 6, wobei die erste reflektierende Oberfläche (120) eine dritte Konfiguration hat zum Ausrichten des Laserstrahls (105) an eine dritte optische Wellenführung (164) in dem Array von optischen Wellenführungen (132).

## Revendications

1. Système de sonde laser ophtalmique comprenant :
un réseau de guides d'onde optiques (132) ;
un adaptateur (102) pouvant fonctionner pour se raccorder avec une source laser (104) ;
une première surface réfléchissante (120) à l'intérieur de l'adaptateur, la première surface réfléchissante étant mobile autour d'un premier axe ; et
une seconde surface réfléchissante (122) à l'intérieur de l'adaptateur, la seconde surface réfléchissante étant mobile autour d'un second axe sensiblement orthogonal par rapport au premier axe,
dans lequel la première surface réfléchissante (120) est configurée pour recevoir un faisceau laser (105) émis depuis la source laser et rediriger le faisceau laser vers la seconde surface réfléchissante (122).

2. Système de sonde laser selon la revendication 1, comprenant en outre un miroir micro-électromécanique qui comprend la première surface réfléchissante (120).

3. Système de sonde laser selon la revendication 1, comprenant en outre une lentille à gradient d'indice (118) à l'intérieur de l'adaptateur (102), la lentille à gradient d'indice étant configurée pour recevoir le faisceau laser (105) émis depuis la source laser et diriger le faisceau laser vers la première surface réfléchissante (120).

4. Système de sonde laser selon la revendication 1, comprenant en outre une lentille à gradient d'indice (124) à l'intérieur de l'adaptateur (102), la lentille à gradient d'indice étant configurée pour recevoir le faisceau laser (105) réfléchi depuis la seconde surface réfléchissante (122).

5. Système de sonde laser selon la revendication 1, dans lequel la première surface réfléchissante (120) a une première configuration pour diriger le faisceau laser (105) vers un premier guide d'onde optique (160) dans le réseau de guides d'onde optiques (132).

6. Système de sonde laser selon la revendication 5, dans lequel la première surface réfléchissante (120) a une deuxième configuration pour diriger le faisceau laser (105) vers un deuxième guide d'onde optique (162) dans le réseau de guides d'onde optiques (132).

7. Système de sonde laser selon la revendication 6, dans lequel la première surface réfléchissante (120) a une troisième configuration pour diriger le faisceau laser (105) vers un troisième guide d'onde optique (164) dans le réseau de guides d'onde optiques (132).
